# EUROPEAN PATENT APPLICATION

(11) **EP 4 233 857 A1**
(43) Date of publication of application: **30.08.2023**
(21) Application number: 23157572.1
(22) Date of filing: 20.02.2023
(51) Int. Cl.: A61K 31/365, A61K 31/522, A61K 31/7076, B08B 17/00, C12N 1/20, A61P 35/00

(54) **BIOACTIVE COMPOUNDS OBTAINED FROM CYANOBACTERA LEPTOTHOE SP. LEGE 181152**

(30) Priority: 28.02.2022 PT 2022117824
(71) Applicant: CIIMAR - Centro Interdisciplinar de Investigação Marinha e Ambiental, 4455-208 Lavra (PT); Universidade Do Porto, 4099-002 Porto (PT); Instituto de Engenharia e Ciências do Mar, Universidade Técnica do Atlântico, 4450-208 Matosinhos (PT)
(72) Inventor: ALVES REIS, MARIANA, 4450-208 MATOSINHOS (PT); REIS ALMEIDA, JOANA, 4450-208 MATOSINHOS (PT); VASCONCELOS, VITOR, 4450-208 MATOSINHOS (PT); PEREIRA MORAIS, JOÃO CARLOS, 4450-208 MATOSINHOS (PT); FERREIRA, LEONOR, 4450-208 MATOSINHOS (PT); PEREIRA, SANDRA, 4450-208 MATOSINHOS (PT); GONÇALVES, CATARINA, 4450-208 MATOSINHOS (PT); NEVES, JORGE, 4450-208 MATOSINHOS (PT)
(74) Representative: Neves, Ana

(57) **Abstract**

The present invention relates to bioactive compounds obtained from the cyanobacterium strain *Leptothoe sp.* LEGE 181152. The compounds obtained from the organic extract of this cyanobacteria are hypoxanthine arabinoside, 2'-deoxyinosine, and phormidolide D.

These compounds were found to have cytotoxic activity towards cancer cells. Additionally, compounds now disclosed also have antifouling activity against macro- and microfouling marine organisms.

## Description

### Technical field

The present disclosure relates to natural compounds that can be obtained from cyanobacterial strain *Leptothoe sp.* LEGE 18115. In particular, the compounds hypoxanthine arabinoside, 2'-deoxyinosine, and phormidolide D.

The compounds now disclosed have cytotoxic activity towards cancer cells.

The compounds now disclosed also have antifouling activity against macro- and microfouling marine organisms (for example mussels and marine biofilm-forming bacteria).

### Background art

Cyanobacteria are a diverse group of photosynthetic prokaryotes, known to produce secondary metabolites with promising bioactivities (Khalifa et al., 2021). Compounds isolated from these organisms have been described for their anticancer (Sousa et al., 2020), anti-inflammatory (Demay, 2021), antibacterial (Cepas et al., 2021), antifouling (Antunes, 2019) and lipid reducing activities (Freitas et al., 2019), among others. These promising results push forward novel biotechnological applications to deliver new chemotherapeutic agents to aid cancer treatment and novel strategies to combat the economic and ecological burden of marine biofouling (Dahms et al., 2006).

Cancer is a group of diseases in which abnormal cells grow uncontrollably and can spread to other organs and tissues. It is one of the main causes of mortality worldwide. In 2020, more than 19 million new cases of cancer were diagnosed, and 10 million deaths were reported. It is predicted that by 2040 the number of new cases will increase by 470 (Sung et al.,2021). Among the most common types of cancer, colorectal cancers were the third most diagnosed cancer in 2020, and the second most deadly (https://gco.iarc.fr/).

Chemotherapy is one of the most common treatments of cancer, this systemic treatment uses drugs to kill fast growing cells. Despite the beneficial effects, chemotherapy still entails multiple negative side effects, as well as high risk of development of drug resistance (Bukowski et al., 2020). These obstacles for a successful cancer treatment, result in a higher mortality rate and in an increase of the socioeconomic burden of this disease. Therefore, it is decisive to search for alternatives that overcome these problems. Natural products, more concretely those produced by cyanobacteria, are an alternative with proven results, as there are currently four FDA-approved drugs used as antibody drug conjugates in the treatment of lymphomas, bladder cancer and myeloma (van de Donk et al., 2012; Hanna, 2020; Deeks, 2019; Tzogani et al., 2021). Nevertheless, due to the importance and strain of cancer to our society, it is a priority to continue to find safer options for treating such a burdensome disease.

Marine biofouling, resulting from the accumulation of microorganisms (e.g. marine bacteria, diatoms, and fungi) and macroorganisms (e.g. macroalgae, sponges, cnidarians, polychaetes, mollusks, barnacles, bryozoans and tunicates) on marine submerged structures, is one of the major problems faced by maritime industries.

Technical and economic issues as a consequence of biofouling includes corrosion of materials and the increase in fuel consumption. (Amara et al., 2018) Particularly on ships, it has been estimated that the increase in hydrodynamic drag associated with vessel hull fouling results in fuel consumption increase by as much as 410. (Yusim et al., 2017) Marine biofouling is also associated with environmental and health problems, due to an increase in gas emissions - an extra 300 million tons of fuels producing an estimated 20 million tons/year in additional greenhouse gases (Rosenhahn et al., 2010) - and the spread of invasive species. (Amara et al., 2018).

Biocidal paints containing organotin compounds, namely tributyltin (TBT), were widely used for decades in the maritime industry to prevent biofouling. However, such coatings resulted in toxicity towards marine ecology, which notably led to the ban of TBT-containing coatings by the International Maritime Organisation. (Lejars et al., 2012; Basu et al., 2020) Since then, some booster biocides in combination with copper have been used, but even these compounds have demonstrated toxicity on living organisms. (Kyei et al., 2020) Thus, the development of greener antifouling compounds with environmentally safe characteristics for practical use has been a priority, and research has increased in the field of natural products (Almeida and Vasconcelos, 2015).

### Summary

The present invention relates to a novel compound phormidolide D (3) of formula:

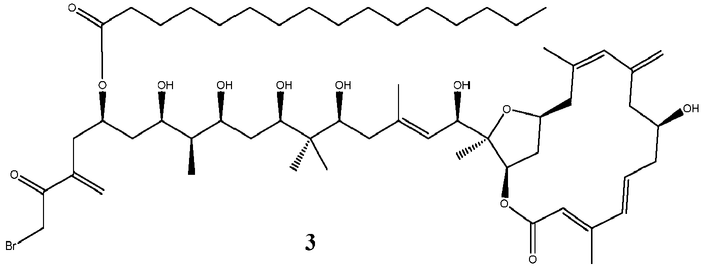

In one embodiment compound phormidolide D (3) is obtained from the organic extract of the cyanobacteria *Leptothoe sp. LEGE 181152.*

In one embodiment compound phormidolide D (3) is for use in the treatment of cancer.

In one embodiment compound phormidolide D (3) is for use in the treatment of colorectal, breast, stomach, prostate, lung, or pancreatic cancer.

The present invention also relates to a composition comprising a therapeutically effective amount of compound phormidolide D (3).

In one embodiment the composition is used in the treatment of cancer.

In one embodiment the composition is used in the treatment of colorectal, breast, stomach, prostate, lung, or pancreatic cancer.

In one embodiment compound phormidolide D (3) is used as an antifouling agent.

The present invention also relates to an antifouling formulation comprising compound phormidolide D (3).

In one embodiment the antifouling formulation is for use in the reduction or prevention of macro- and microfouling marine organisms' attachment.

The present invention also relates to bioactive compounds obtained from the organic extract of the cyanobacteria Leptothoe sp. LEGE 181152, wherein the compounds are hypoxanthine arabinoside (1) and 2'-deoxyinosine (2).

In one embodiment the bioactive compound hypoxanthine arabinoside (1) is for use as antifouling agent.

In one embodiment the bioactive compound 2'-deoxyinosine (2) is for use as antifouling agent.

The present invention also relates to an antifouling formulation comprising compound hypoxanthine arabinoside (1) or compound2'-deoxyinosine (2).

In one embodiment the antifouling formulation is for use in the reduction or prevention of macro- and microfouling marine organisms.

### General description

The present disclosure relates to cyanobacterial natural compounds, with activity towards cancel cells, for example spheroids of the human colorectal carcinoma cell line (HCT 116) .

The same cyanobacterial natural products including fractions and compounds also have antifouling activity against macrofouling (for example, *Mytilus galloprovincialis* plantigrades) and microfouling (for example, *Roseobacter litoralis* biofilm-forming bacteria) marine organisms.

These natural compounds have the advantage that they can be obtained from a natural resource. The cyanobacterial strain *Leptothoe sp.* LEGE 181152, is commercially available and can be used to obtain these cytotoxic and antifouling natural products.

The compounds, in particular hypoxanthine arabinoside, 2'-deoxyinosine, and phormidolide D, of the presented disclosure were obtained through bioassay guided approaches.

The hypoxanthine arabinoside and 2'-deoxyinosine are commonly known components of the nucleic acid's building blocks. Their biological potential resides on their activity as antiviral agents, as it has been documented in literature (Bryson & Connor, 1976; Falcon et al., 1977; Novotny et al., 2000), and expressed in patent documents such as US2013281362A1, WO2012096596A1, US2008249066A1 and US6653081B2. However, antifouling activity, either towards micro and/or macrofouling, is not described to date in the literature for these compounds.

The novel cytotoxic compound phormidolide D was not described to date in the literature. Another compound with similar structural identity, phormidolide A, was reported as noncytotoxic against a panel of 60 cancer cells, despite its toxicity to brine shrimp, with a LD50 of 1.5 µM (Williamson et al., 2002). Other compounds from the phormidolide family phormidolide B and C, have been described for their cytotoxic activity against three cell lines, Lung (A-549), colon (HT-29) and breast (MDA-MB-231), with IC₅₀ values around 0.8 to 1.4 µM (Lorente et al., 2014). Although belonging to the same family, they are structurally different from the new phormidolide D.

### Brief description of drawings

For easier understanding of this application, figures are annexed that represent the preferred forms of implementation which nevertheless are not intended to limit the technique disclosed herein.
Figure 1 - Cytotoxic assessment of 8 VLC fractions from cyanobacterium *Leptothoe sp.* LEGE 181152 on HCT 116 spheroids (at 25 µg/mL) in comparison with solvent control (DMSO) and staurosporine: (A) percentage of cell viability; (B) bright field images of HCT 116 spheroids. " 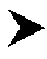 " indicates the spheroid well-defined exterior boundary and "*" indicates the spheroid necrotic core. Staurosporine was tested at 1.25 µM.
Figure 2 - Dose-response cytotoxic effects of the active fractions F+G1 and G2 (A) and the corresponding bright field images (B) of HCT 116 spheroids. " 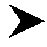 " indicates the spheroid well-defined exterior boundary and "*" indicates the spheroid necrotic core. Staurosporine was tested at 1.25 µM.
Figure 3 - Anti-settlement assay performed on the 8 VLC fractions towards *Mytilus galloprovincialis plantigrades,* at a concentration of 10 µg/mL. Negative control (C-): filtered seawater; Positive control (C+): CuSO₄.
Figure 4 - Schematic representation of the bioactivity-guided fractionation of *Leptothoe sp.* LEGE 181152 methanolic extract.
Figure 5 - Cell viability of the spheroids from the HCT 116 cell line after exposure to subfractions A - K from G2_8+9 at a concentration of 30 µg/mL. DMSO was used as solvent control and staurosporine was positive control (tested at 2.5 µM). Percentage of cell viability was calculated after normalisation to solvent control.
Figure 6 - Anti-settlement assay performed on fractions A-K from G2_8-9, towards *Mytilus galloprovincialis plantigrades,* at a concentration of 10 µg/mL. Negative control (C-): filtered seawater; Positive control (C+): CuSO₄.
Figure 7 - Growth inhibition of *Roseobacter litoralis* when incubated with the subfractions G2_8+9-A - K, at a concentration of 100 µg/mL. Control -: 1 % DMSO; Control +: 1:100 dilution of penicilin-streptomycin-neomycin stabilised solution (Sigma P4083).
Figure 8 - Anti-settlement assay performed on fractions A-E from the flash chromatography of subfraction 11, towards *Mytilus galloprovincialis plantigrades,* at a concentration of 10 µg/mL. Negative control (C-): filtered seawater; positive control (C+): CuSO₄.
Figure 9 - Structures of compound hypoxanthine arabinoside (1) and compound 2'-deoxyinosine (2).
Figure 10 - Structure of compound phormidolide D (3).
Figure 11 - Dose-response cytotoxic effects of phormidolide D on the monolayer model of HCT 116 cells (A) and on the 3D spheroids (B) with the corresponding bright field images (C). Staurosporine was tested at 1.25 µM.

### Description of embodiments

Now, preferred embodiments of the present application will be described in detail with reference to the annexed drawings. However, they are not intended to limit the scope of the present disclosure.

The present disclosure relates to bioactive fractions and compounds, obtained from the organic extract of the cyanobacterium *Leptothoe sp.* LEGE 181152.

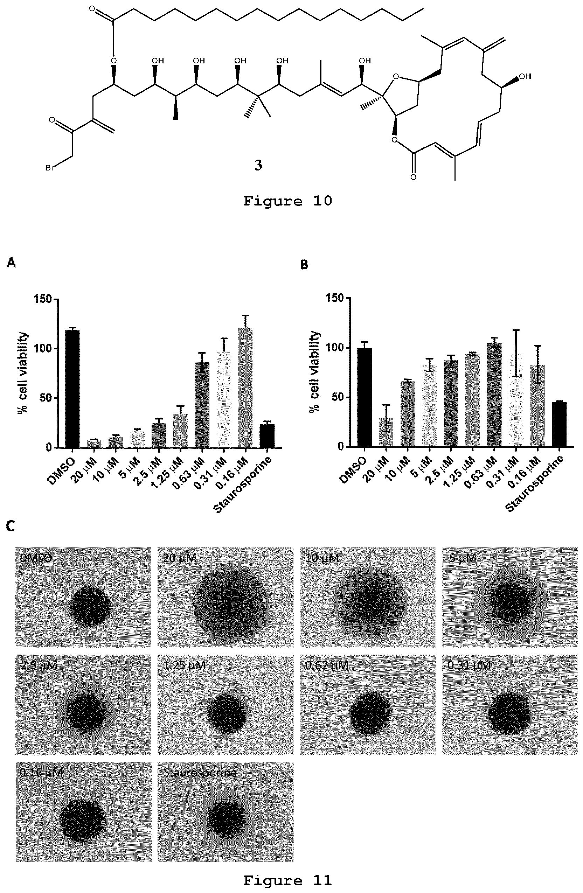

The present application discloses the novel compound phormidolide D (3) as shown in Figure 10.

In one embodiment, compound phormidolide D (3) is obtained from the organic extract of the cyanobacteria *Leptothoe sp. LEGE 181152.*

Compound phormidolide D (3) can be used in the treatment of cancer. In one embodiment the cancer is colorectal, breast, stomach, prostate, lung, or pancreatic cancer.

The present application also relates of a pharmaceutical composition comprising a therapeutically effective amount of compound phormidolide D (3). In one embodiment, the pharmaceutical composition is used in the treatment of cancer. In one embodiment the cancer is colorectal, breast, stomach, prostate, lung, or pancreatic cancer.

The present application also relates to compounds hypoxanthine arabinoside (1) and 2'-deoxyinosine (2) obtained from the organic extract of the cyanobacteria *Leptothoe sp. LEGE 181152.*

Compounds hypoxanthine arabinoside (1) and 2'-deoxyinosine (2) can be used as antifouling agents.

Compound phormidolide D (3) can also be used as an antifouling agent.

The present application also relates to an antifouling formulation comprising compound hypoxanthine arabinoside (1), an antifouling formulation comprising compound 2'-deoxyinosine (2) and an antifouling formulation comprising compound phormidolide D (3).

In one embodiment, the antifouling formulation is used in the reduction or prevention of macro- and microfouling marine organisms.

In one embodiment the marine organisms are *Mytilus galloprovincialis* plantigrades or *Roseobacter litoralis.*

### Examples

This organism was isolated from an intertidal cyanobacterial mat collected in Baía das Gatas (São Vicente Island), Cape Verde (16°54'11.2"N 24°54'16.9"W) and is maintained at the LEGEcc in CIIMAR, Matosinhos, Portugal (the strains were commercially purchased).

### Method

The strain was cultured in Z8 medium supplemented with 25‰ of synthetic sea salts (Tropic marin, Germany) and 1‰ of vitamin B12 at 25 °C, with a photoperiod of 14 h/10 h light and dark respectively, and at light intensity of 10-30 µmols photons s⁻¹m⁻². Cultures were grown up to 40 L with constant aeration and at the exponential phase, cells were harvested, frozen and freeze-dried. The biomass of *Leptothoe sp.* LEGE 181152 (36.3 g) was extracted with 4.75 L of methanol yielding crude extract of 12.5 g. To concentrate the chemical components in different polarities, the crude was subjected to a vacuum liquid chromatography using as stationary phase 360 g of silica gel-60, and as mobile phase a step gradient of hexane/ethyl acetate (0.5 L each mixture), and mixtures of ethyl acetate/methanol (0.5 L each). This chromatography yielded 10 fractions (Table 1). The chemical composition of the fractions was evaluated by thin layer chromatography (TLC), and since fractions F and G1 showed similar TLC profiles, they were pooled together and denominated as fraction F+G1.

**Table 1. Fractions of the methanol extract of Leptothoe sp. LEGE 181152, their polarity and mass**

| **Fraction** | **Polarity** | | **Mass (mg)** |
|---|---|---|---|
| | Hexane: ethyl acetate | Ethyl acetate: methanol | |
| A | 4:1 | - | 146,86 |
| B | 7:3 | - | 48,66 |
| C | 3:2 | - | 370,68 |
| D | 2:3 | - | 296,57 |
| E | 1:4 | - | 36,48 |
| F | 0:1 | - | 126,26 |
| G1 | - | 7:3 | |
| G2 | - | 7:3 | 1675,6 |
| H1 | - | 0:1 | 243,01 |
| H2 | - | 0:1 | 3435,87 |

### Cytotoxicity activity against cancer cells

The cytotoxicity of the fractions A to H2 was tested against the 3D model of the human colorectal carcinoma cell line, HCT 116. Colorectal cancer has one of the highest incidences among cancer diagnosis and is also one of cancers with the highest mortality rate. Over the years, the 3D cell models have taken over drug screening due to their biologically relevance when compared to the more conventional monolayer model. Three dimensional spheroids are more representative of the microenvironment of an *in vivo* tumour, with interactions between cells in the different layers - a necrotic core, an intermediate quiescent layer, and a proliferative outer layer. The 3D structure also provides a gradient of different concentrations of oxygen and nutrients, as well as for drug penetration so it is a great model for predicting the behaviour of drug response in a solid tumour using in vitro models, making drug screening more effective.

Cells were seeded in ULA 96 well plates and incubated for 5 days for spheroid formation. After formation, spheroids were exposed to the fractions (25 µg/mL) for 96h. Cell viability was assessed by the acid phosphatase assay. In viable cells these intracellular enzymes hydrolyse p-nitrophenol, which can be quantified colorimetrically at 405 nm. The results were expressed as the percentage of cell viability normalized to the solvent control (DMSO).

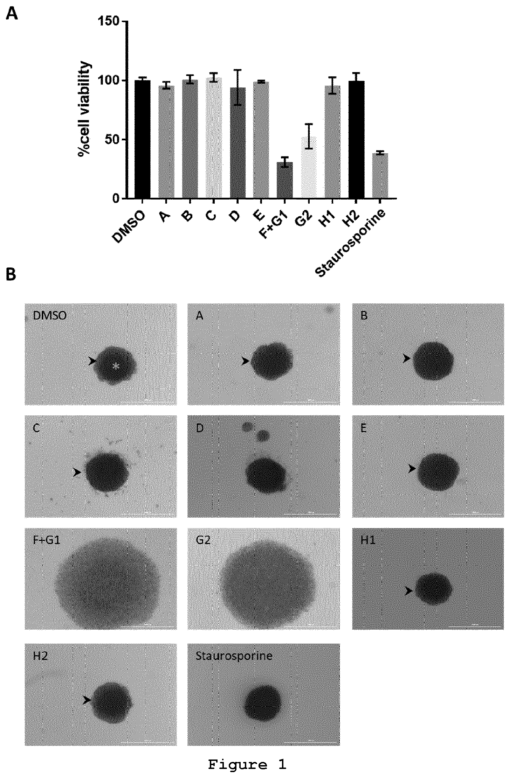

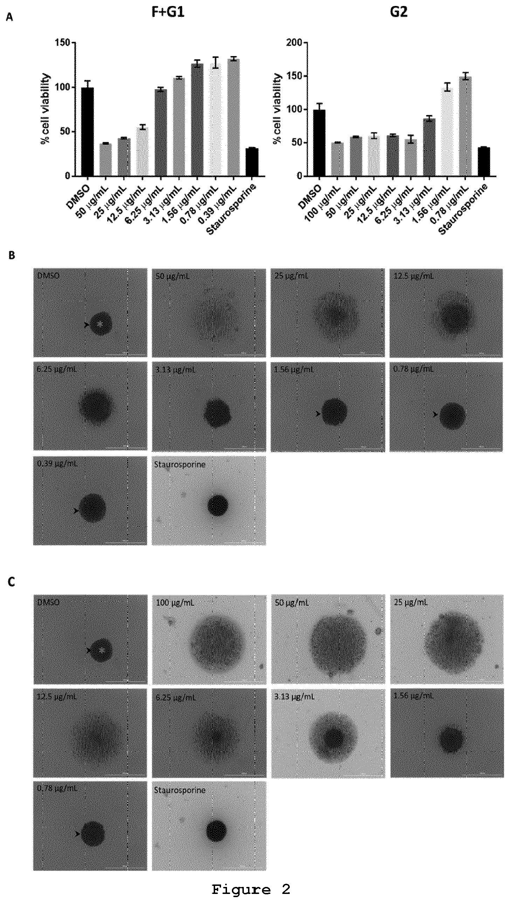

Figure 1A shows the effect of the fractions A to H2 on the viability of the cancer spheroids and it is noticeable an evident cytotoxic effect of fractions F+G1 and G2, representing a reduction of cellular viability respectively of 69% and 47%. These observations indicate that the chemical composition of fractions F+G1 and G2 is able to reduce the viability of HCT 116 cancer cells, an effect comparable to staurosporine, which is a reference anticancer compound known to induce apoptosis in a variety of cancer cells. Figure 1B shows the effects of fractions A to H2 on the spheroid morphology. Viable spheroids appear with a well-defined exterior boundary ( 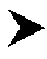 ) and a darker core (*). Whereas in the ones exposed to fractions F+G1 and G2 it can be seen that the morphology was considerably affected, with loss of spheroid differentiation, and with increase of volume and diameter. This cytotoxic effect was different to what was observed for staurosporine, suggesting the active fractions F+G1 and G2 to induce cell death through a different molecular mechanism. Figure 2 shows the dose-dependent cytotoxic effect of fractions F+G1 and G2 on the colon cancer HCT 116 spheroids. The calculated inhibitory concentration that reduces cell viability to 500 (IC₅₀) is 23.04 ug/mL for fraction F+G1 and 58.23 µg/mL G2.

### Antifouling activity

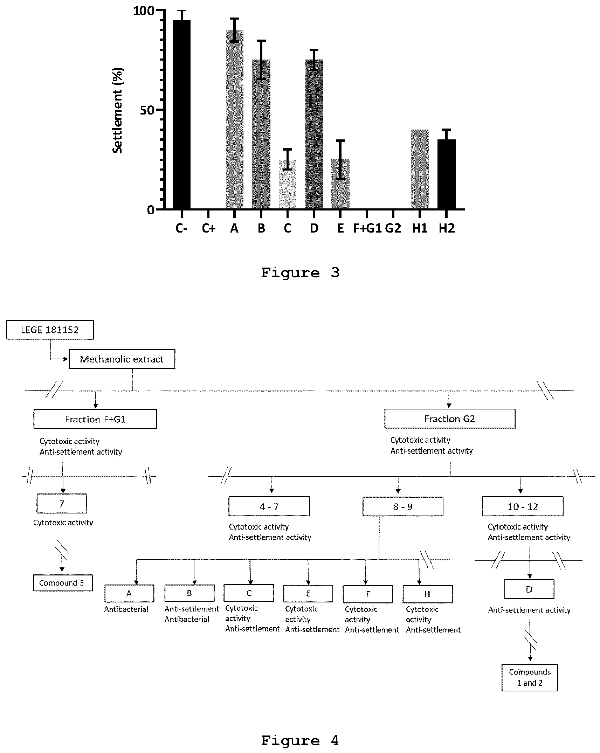

The same VLC fractions A to H2 (Table 1) were assessed for their potential antifouling activity using an anti-settlement bioassay towards *Mytilus galloprovincialis* plantigrades, the adhesive stadium of mussel larval cycle. Results showed anti-settlement activity, particularly in fraction F+G1 and G2 with 100 % of inhibition of the mussel plantigrades, at a concentration of 10 ug/mL (Figure 3).

Since the fractions F+G1 and G2 showed the advantage of having two distinct beneficial applications either as cytotoxic against cancer cells or as antifouling activity, further bioactivity-guided fractionation was performed (Figure 4).

### Study of fraction G2_8-9

A flash chromatography (Pure C-850 FlashPrep, Buchi) was performed to fraction G2 (1.6756 g), using as stationary phase SiO₂ (Silicycle 25 g cartridge), and as mobile phase mixtures of hexane: ethyl acetate (1:1 to 0:1) and ethyl acetate: methanol (1:0 to 0:1). This chromatography yielded 15 fractions that were pooled according to their TLC profile. Fractions G2_4-7 (39.85 mg), G2_8-9 (744 mg) and G2_10-12 (160.38 mg) showed activity in the cytotoxic and anti-settlement assays (Figure 4). Only fractions G2_8-9 and G2_10-12 were further fractionated, due to the available amounts.

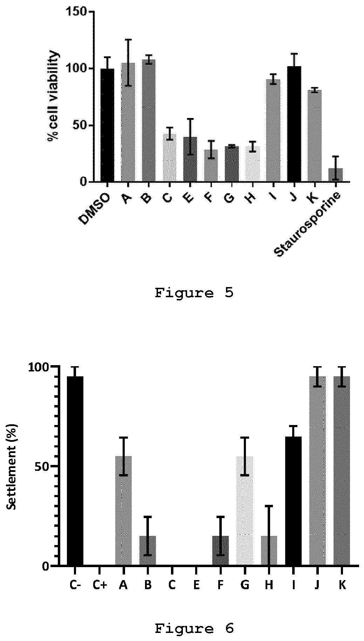

Thus, fraction G2_8-9 was submitted to a flash chromatography (Pure C-850 FlashPrep, Buchi) with a reverse-phase C-18 column (40 g) using a mixture of methanol: water (2:3 to 1:0), from which resulted subfractions A - K. These fractions were tested for their cytotoxic potential on the 3D HCT 116 cell spheroids at a concentration of 30 µg/mL for 96 h (Figure 5). Fractions C, E-H were able to reduce cell viability from 60% to 70%. Fraction D was not tested due to its small amount.

According to the anti-macrofouling screening through the anti-settlement assay, subfractions B, C, E, F and H presented an inhibition of mussel larvae adhesion between 85% to 100%, at a concentration of 10 ug/mL (Figure 6).

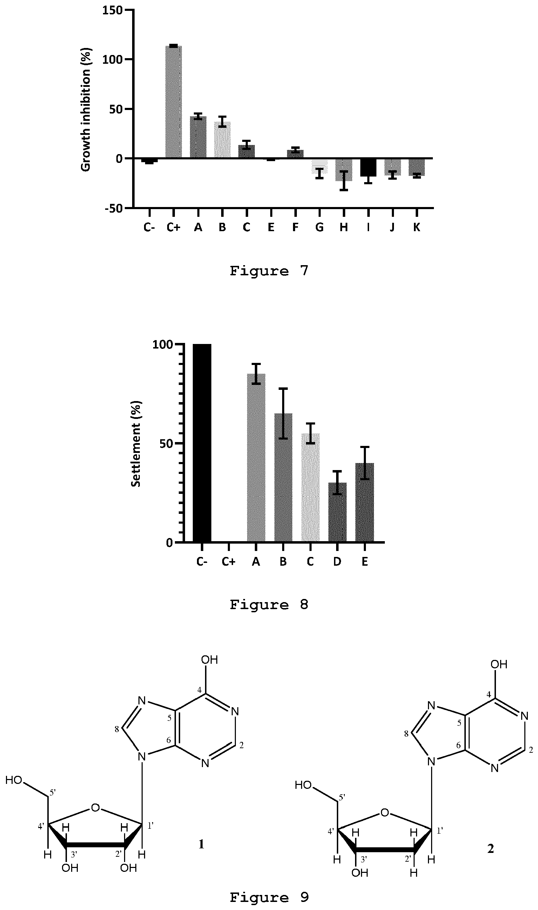

Considering that microfouling is responsible for the maturation of biofilms, which may later enable the settlement of larvae and spores from macrofouling organisms like *Mytilus galloprovincialis*, the assessment of antifouling potential towards biofilm-forming bacteria is also valuable. Hence, fractions G2_8-9_A-K were tested in the growth inhibition assay against five biofilm-forming marine bacteria *Cobetia marina, Halomonas aquamarina, Pseudoalteromonas atlantica, Roseobacter litoralis* and *Vibrio harveyi.* Results showed inhibitory activity against marine bacteria *Roseobacter litoralis* growth, particularly induced by fractions A and B with an inhibition higher than 300, at a concentration of 100 ug/mL (Figure 7).

### Study of fraction G2_10-11

A flash chromatography (Pure C-850 FlashPrep, Buchi) was performed on fraction G2_10-12 using a SiliaSep 4 g cartridge (Silicycle) and by eluting the sample in isopropyl alcohol: ethyl acetate (from 0:1 to 1:0, in increasing gradients of 10 %). This chromatography yielded 5 subfractions A-E that were screened in the anti-settlement assay, and G2_10-12_D (41.14 mg) was active against mussel plantigrades, with a settlement inhibition of 70 %, at 10 µg/mL.

Fraction G2_10-12_D was separated on a SPE C-18 cartridge (Strata^{®} C18-E 55 um, 70 A, 1g/6 mL) using a gradient starting with a mixture of methanol: water (1:1, 12 mL), then methanol (12 mL), and then dichloromethane (12 mL), originating two fractions G2_10-12_D1 (33.88 mg) and G2_10-12_D2 (6.12 mg). Fraction G2_10-12_D1 was purified by HPLC using a reverse-phase C18-AR column (ACE Excel 3 µm C18-AR, 75 × 4.6 mm) with acetonitrile: water in a gradient from 1:100 to 1:20 in 20 min (1 ml/min), yielding 1.85 mg of compound **1** (Rt = 10.07 min) and 0.84 mg of compound **2** (Rt = 11.45 min).

Compound **1** (Figure 9) was identified as hypoxanthine arabinoside by comparing its spectroscopic data with the literature data. The ESIMS spectrum of compound **1** presented a protonated molecule at m/z 268 [M+H]⁺, consistent with the molecular formula C₁₀H₁₂N₄O₅. This was further corroborated through NMR experiments, namely 1D (¹H and ¹³C) and 2D (¹H-¹H COSY, HBMC, HSQC) that revealed the compound **1** to be a nucleoside.

The ¹³C, HSQC and HMBC data confirmed the purine moiety to be hypoxanthine in the enol form [δc 157.6 (C-4), 153.5 (C-2), 150.0 (C-6), 142.0 (C-8), 121.0 (C-5)]. Moreover, the ¹H and ¹³C resonances of the glycosidic residue were comparable to those of the arabinofuranoside of inosine (Brillatz et al., 2018).

NMR assignments: ¹H NMR (400.14 MHz, MeOD): δ 8.34 (1H, s, H-8), 8.22 (1H, s,H-2), 6.00 (1H, d, *J* = 6.4 Hz, H-1'), 4.75 (1H, m, H-2'), 4.36 (1H, dd, *J* = 5.1, 2.6 Hz, H-3'), 4.20 (1H, q, *J* = 2.6 Hz, H-4'), 3.90 (1H, dd, *J* = 12.5, 2.5 Hz, H-5a'), 3.78 (1H, dd, *J* = 12.6, 2.7 Hz, H-5b') ppm.

¹³C NMR (100.63 MHz, MeOD): δ 157.6 (C-4), 153.5 (C-2), 150.03 (C-6), 142.0 (C-8), 121.0 (C-5), 91.2 (C-1'), 88.1 (C-4'), 75.4 (C-2'), 72.6 (C-3'), 63.4 (C-5') ppm.

Compounds **1** and **2** (Figure 9) presented similar proton spectra, and through the analysis of their ESIMS a difference 16 mass units was observed. Given the [M+H]⁺ ion at *m*/*z* 252, it could be inferred that compound **2** lacks an oxygen atom in comparison to **1,** which is consistent with the molecular formula C₁₀H₁₂N₄O₄. This observation was ascertained with the ¹H NMR spectrum, which revealed the difference of the molecules to be in the sugar moiety. Namely, the multiplicity of the signal of the anomeric proton changed from a doublet in compound **1** [6.00 (d, *J* = 6.4 Hz)] to doublet of doublets at δ_{H} 6.43 (dd, *J* = 8.0, 6.0 Hz) in **2.** Moreover, the proton signals of position 2 appeared as two diastereotopic protons at δ_{H} 2.81 (ddd, *J* = 13.6, 8.0, 5.8 Hz, H-2a') and 2.42 (ddd, *J* = 13.6, 6.0, 2.7 Hz, H-2b') confirming the loss of the hydroxyl group [compound **1** δ 4.75 (m, H-2')] at this position. Thus, the glycosidic part was identified as a 2'-deoxyribose and the compound **2** was identified as 2'-deoxyinosine.

NMR assignments: ¹H NMR (400.14 MHz, MeOD): δ 8.34 (1H, s, H-8), 8.20 (1H, s, H-2), 6.43 (1H, dd, *J* = 8.0, 6.0 Hz, H-1'), 4.08 (1H, q, *J* = 3.0 Hz, H-4'), 3.85 (1H, dd, *J* = 12.3, 3.0 Hz, H-5a'), 3.75 (1H, dd, *J* = 12.4, 3.3 Hz, H-5b'), 2.81 (1H, ddd, J = 13.6, 8.0, 5.8 Hz, H-2a'), 2.42 (1H, ddd, J = 13.6, 6.0, 2.7 Hz, H-2b') ppm.

### Study of subfraction F+G1

Subfraction F+G1 was submitted to a flash chromatography (Pure C-850 FlashPrep, Buchi), using a SiliaSep 24 g (Silicycle) cartridge as stationary phase with a gradient of hexane and ethyl acetate (9:1 to 0:1), yielding 11 fractions from which subfraction F+G1_7, with 70.78 mg, was chosen for purification. Subfractionation was performed by HPLC in a semi-preparative C18-AR column (ACE 10 C18-AR, 250 × 10 mm) with a isocratic elution of acetonitrile:water (9:1, 5ml/min) for 30 min to obtain 15.3 mg of fraction F+G1_7_A (Rt = 10.93 min). This was further chromatographed using the same column with an isocratic elution of methanol:water (9:1, 5ml/min, 24 min) and then the fraction obtain at 16.19 min (4.61 mg) was finally purified using an analytic column (ACE Excel 3 um C18-AR, 75 × 4.6 mm) with acetonitrile:water (4:1, 1mL/min, 15 min), yielding 1.5 mg of compound **3** (Figure 10).

The molecular formula C₅₈H₉₅BrO₁₂, consistent with 11 degrees of unsaturation, was deduced from HRESIMS at *m*/*z* 1061.5947 [M - H]⁻ (calculated for C₅₈H₉₄BrO₁₂ 1061.5929). The ¹H and ¹³C NMR data suggested compound **3** to be a polyketide related to phormidolide A (Williamson et al, 2002). The planar structure of compound **3** was fully assigned by 1D and 2D NMR experiments (COSY, HSQC and HMBC) and through comparison of phormidolide A spectroscopic data. Table 2 presents the complete NMR assignments of compound **3.** The difference between these compounds is at the positions C32-C33, in which for phormidolide A, these positions belong to an olefinic bond (C32 δ_{c} 158.4; C33 δ_{c} 78.8). While in compound **3**, C32 presents a low field carbonyl signal at δ_{c} 192.6 ppm, typical of conjugated ketones, and C33 corresponds to a terminal methylene bromide (δ_{c} 30.7). The presence of the ketone function at this position was confirmed by the HMBC correlations between C32 and the olefinic protons (H₄₂ₐ δ 5.02 s and H_{42b} δ 4.86 s) of the exo-methylene group Δ31(42), and the protons of the terminal methylene bromide group [H₃₃ₐ δ 4.27 (d, *J* = 12.2 Hz) and H_{33b} δ 4.18 (d, *J* = 12.2 Hz)]. As two other phormidolides, B and C (Lorente *et al.,* 2014), had been previously described, compound **3** was named as phormidolide D. The stereochemistry of **3** was assumed to be the same as phormidolide A (Ndukwe *et al,* 2020) through the comparison of proton and carbon chemical shifts and proton coupling constants.

**Table 2. ¹H NMR (600.13 MHz) and ¹³C NMR (150.9 MHz) spectroscopic data (δ in ppm) for compound 3**

| **Position** | **δC, type** | **δH, (*J* in Hz)** |
|---|---|---|
| 1 | 167.6, C | |
| 2 | 118.4, CH | 5.8, s |
| 3 | 152.4, C | |
| 4 | 134.3, CH | 6.2, d (15.4) |
| 5 | 132.7, CH | 5.89, m |
| 6a | 44.3, CH₂ | 2.86, bd (12.2) |
| 6b | | 2.14, bd (1.4) |
| 7 | 73.63, CH | 4.04, ovlp |
| 8a | 44.04, CH₂ | 2.48, ovlp |
| 8b | | 1.84, ovlp |
| 9 | 141.6, C | |
| 10 | 132.6, CH | 5.29, s |
| 11 | 133.6 | |
| 12a | 48.4, CH₂ | 2.60, dd (13.2, 4.8) |
| 12b | | 2.34, ovlp |
| 13 | 76.8, CH | 4.49, ovlp |
| 14a | 34.9, CH₂ | 2.32, ovlp |
| 14b | | 1.56, ovlp |
| 15 | 79.8, CH | 5.16 d (4.5) |
| 16 | 87.2, C | |
| 17 | 70.0, CH | 4.72, d (8.9) |
| 18 | 127.4, CH | 5.44, d (8.9) |
| 19 | 137.5, C | |
| 20a | 42.3, CH₂ | 2.38, ovlp |
| 20b | | 2.06, dd (13.3, 10.8) |
| 21 | 77.9, CH | 3.66, bs |
| 22 | 40.6, C | |
| 23 | 81.9, CH | 3.86, bd (11.5) |
| 24a | 35.2, CH₂ | 1.65, m |
| 24b | | 1.47, ovlp |
| 25 | 78, CH | 4.07, bd (10.4) |
| 26 | 41.5, CH | 1.45, ovlp |
| 27 | 73.61, CH | 3.98, t (6.4) |
| 28 | 39.2, CH | 1.82, ovlp |
| 29 | 70.3, CH | 5.03, m |
| 30a | 37.1, CH₂ | 2.79, dd (13.9, 3.8) |
| 30b | | 2.43, m |
| 31 | 142.6, C | |
| 32 | 192.6, C | |
| 33a | 30.7, CH₂ | 4.27, d (12.3) |
| 33b | | 4.18, d (12.3) |
| 34 | 14.1, CH₃ | 2.10, s |
| 35a | 114, CH₂ | 4.98, s |
| 35b | | 4.77, s |
| 36 | 17.0, CH₃ | 1.58, s |
| 37 | 21.3, CH₃ | 1.19, s |
| 38 | 17.7, CH₃ | 1.81, s |
| 39 | 13.7, CH₃ | 0.91, s |
| 40 | 21.7, CH₃ | 0.75, s |
| 41 | 5.1, CH₃ | 0.94, d (7.1) |
| 42a | 128.5, CH₂ | 6.08, s |
| 42b | | 5.93, s |
| 43 | 173.8, C | |
| 44 | 34.7, CH₂ | 2.23/2.32, ovlp |
| 45 | 25.1, CH₂ | 1.58/1.58, ovlp |
| 46-58 | 29.9, CH₂ | 1.25/1.25, ovlp |
| 59 | 14.3, CH₃ | 0.88, t (6.9) |

### Cytotoxic activity against cancer cells

The cytotoxic effect of phormidolide D (**3**) was tested against HCT 116 human colon carcinoma cells in monolayer (2D) and spheroid models (Figure 11). The IC₅₀ of the compound was 1.21 µM for the 2D assay and 13.58 µM in cancer spheroids, and thus can be considered an active cytotoxic agent (Indrayanto et al., 2020). The difference of the IC₅₀ values between the two cell models is commonly reported in the literature. Cancer spheroids tend to be more resistant to treatment than monolayer cells, and thus tend to present higher IC₅₀ values. However, the cytotoxic effects in cancer spheroids are more realistic, because in this culture model, drug diffusion and cell proliferation effects are being tested. The results obtained for compound **3** are very interesting: despite its molecular weight (1064 g/mol) it can still penetrate the tightly packed cells of the outer layer of the spheroid, leading to a decrease in the cell viability in relevant concentrations (Figure 9). It is important to note that phormidolide A was not considered active against a panel of 60 human cancer cell lines used in the U.S. National Cancer Institute (Williamson et al, 2002). Since phormidolide A and D share the same scaffold, the cytotoxicity against HCT 116 cancer cells might be explained by the presence of the keto form with methylene bromide group in phormidolide D (3), instead of the methyl-trapped enol ether with vinyl bromide functionality of phormidolide A. Cytotoxic activity against cancer cells was also reported for phormidolide B and C (Lorente et al., 2014), however these compounds are different from phormidolide D (3) as: they have a different macrolide core (14-membered lactones vs 16-membered lactone of compound (3); the macrolide core presents different unsaturation and substitution pattern; the polyol chain is similar to phormidolide A; and the fatty acid presents different size (C14 vs C16 of compound 3), substitution pattern and degree of unsaturation.

A MS analysis to the fraction F+G1_7, showed the main components to be phormidolide D (3) and A, phormidolide B and C were not detected in the sample. Therefore, the cytotoxic activity of fraction F+G1_7 can be attributed to phormidolide D (3).

This description is of course not in any way restricted to the forms of implementation presented herein and any person with an average knowledge of the area can provide many possibilities for modification thereof without departing from the general idea as defined by the claims. The preferred forms of implementation described above can obviously be combined with each other. The following claims further define the preferred forms of implementation.

### References

Almeida, J. R.; Vasconcelos, V. Natural antifouling compounds: Effectiveness in preventing invertebrate settlement and adhesion. Biotechnol Adv, 2015, 33 (3-4), 343-57.
Amara, I.; Miled, W.; Slama, R. B.; Ladhari, N., Antifouling processes, and toxicity effects of antifouling paints on marine environment. A review. Environ Toxicol Pharmacol, 2018, 57, 115-130.
Antunes, J.; Pereira, P.; Ribeiro, T.; Plowman, J. E.; Thomas, A.; Clerens, S.; Campos, A.; Vasconcelos, V.; Almeida, J. R.; A Multi-Bioassay Integrated Approach to Assess the Antifouling Potential of the Cyanobacterial Metabolites Portoamides. Marine Drugs, 2019, 17, 111. https://doi.org/10.3390/md17020111
Basu, S.; Hanh, B. M.; Isaiah Chua, J. Q.; Daniel, D.; Ismail, M. H.; Marchioro, M.; Amini, S.; Rice, S. A.; Miserez, A., Green biolubricant infused slippery surfaces to combat marine biofouling. J Colloid Interface Sci, 2020, 568, 185-197.
Brillatz, T.; Lauritano, C.; Jacmin, M.; Khamma, S.; Marcourt, L.; Righi, D.; et al. Zebrafish-based identification of the antiseizure nucleoside inosine from the marine diatom Skeletonema marinoi. PLoS ONE 2018, 13 (4), e0196195. https://doi.org/10.1371/journal.pone.0196195 Bryson, Y. J.; Connor, J. D. In Vitro Susceptibility of Varicella Zoster Virus to Adenine Arabinoside and Hypoxanthine Arabinoside. Antimicrobial Agents and Chemotherapy, 1976, 9, 540-543
Dahms, H. U.; Ying, X.; Pfeiffer, C. Antifouling potential of cyanobacteria: a mini-review. Biofouling 2006, 22 (5-6), 317-27.
Deeks, E.D. Polatuzumab vedotin: first global approval. Drugs, 2019, 79 (13), 1467-75.
Demay, J.; Halary, S.; Knittel-Obrecht, A.; Villa, P.; Duval, C.; Hamlaoui, S.; Roussel, T.; Yéprémian, C.; Reinhardt, A.; Bernard, C.; Marie, B. Anti-Inflammatory, Antioxidant, and Wound-Healing Properties of Cyanobacteria from Thermal Mud of Balaruc-Les-Bains, France: A Multi-Approach Study. Biomolecules, 2021, 11 (1), 28. https://doi.org/10.3390/biom11010028
Falcon, M. G.; Jones, B. R. Antiviral Activity in the Rabbit Cornea of Adenine Arabinoside, Ara-A 5' Monophosphate, and Hypoxanthine Arabinoside, and Interactions with Adenosine Deaminase Inhibitor. Journal of General Virology ,1977, 36, 199-202.
Freitas, S.; Silva, N. G.; Sousa, M. L.; Ribeiro, T.; Rosa, F.; Leão, P. N.; Vasconcelos, V.; Reis, M. A.; Urbatzka, R. Chlorophyll Derivatives from Marine Cyanobacteria with Lipid-Reducing Activities. Marine Drugs 2019, 17 (4), 229. https://doi.org/10.3390/md17040229
Hanna, K.S. Clinical overview of enfortumab vedotin in the management of locally advanced or metastatic urothelial carcinoma. Drugs, 2020, 80 (1), 1-7.
Indrayanto, G.; Putra, G. S.; Suhud, F. Validation of invitro bioassay methods: Application in herbal drug research. Profiles of Drug Substances, Excipients and Related Methodology, 2020. doi:10.1016/bs.podrm.2020.07.005
Karol, B.; Kciuk, M.; Kontek, R. Mechanisms of Multidrug Resistance in Cancer Chemotherapy. International Journal of Molecular Sciences, 2020, 21, 3233. https://doi.org/10.3390/ijms21093233
Khalifa, S. A.M.; Shedid, E. S.; Saied, E. M.; Jassbi, A. R.; Jamebozorgi, F. H.; Rateb, M. E.; Du, M.; Abdel-Daim, M. M.; Kai, G-Y.; Al-Hammady, M. A.M.; Xiao, J.; Guo, Z.; El-Seedi, H. R. Cyanobacteria-From the Oceans to the Potential Biotechnological and Biomedical Applications. Marine Drugs, 2021, 19 (5), 241. https://doi.org/10.3390/md19050241
Kyei, S. K.; Darko, G.; Akaranta, O. E., Chemistry and application of emerging ecofriendly antifouling paints: a review. Journal of Coatings Technology and Research, 2020, 17, 315-332.
Lejars, M.; Margaillan, A.; Bressy, C., Fouling release coatings: a nontoxic alternative to biocidal antifouling coatings. Chem Rev, 2012, 112 (8), 4347-90.
Lorente, A.; Gil, A.; Fernández, R.; Cuevas, C.; Albericio, F.; Álvarez, M. Phormidolides B and C, cytotoxic agents from the sea: Enantioselective synthesis of the macrocyclic core. Chem. Eur. J. 2015, 21 (1), 150-156.
Ndukwe, I. E.; et al. Synergism of anisotropic and computational NMR methods reveals the likely configuration of phormidolide A. Chem Comm. 2020, 56 (55), 7565-7568.
Novotny, L.; Abdel-Hamid, M.; Hamza, H., Inosine and 2'-deoxyinosine and their synthetic analogues: lipophilicity in the relation to their retention in reversed-phase liquid chromatography and the stability characteristics. Journal of Pharmaceutical and Biomedical Analysis, 2000, 24, 125 - 132 Rosenhahn, A.; Schilp, S.; Kreuzer, H. J.; Grunze, M., The role of "inert" surface chemistry in marine biofouling prevention. Physical Chemistry Chemical Physics, 2010, 12 (17), 4275-4286.
Sung, 2021 https://doi.org/10.3322/caac.21660
Tzogani, K.; Penttilä, K.; Lähteenvuo, J.; Lapveteläinen, T.; Lopez, A. L., Prieto, C. et al. Ema review of belantamab mafodotin (blenrep) for the treatment of adult patients with relapsed/refractory multiple myeloma. Oncologist, 2021, 26 (1),70-6.
van de Donk, N. W.C.J. and Dhimolea E. Brentuximab vedotin. MAbs. Taylor & Francis, 2012, Vol. 4. No. 4.
Virginio, C.; Gutiérrez-Del-Río, I.; López, Y.; Redondo-Blanco, S.; Gabasa, Y.; Iglesias, M. J.; Soengas, R.; Fernández-Lorenzo, A.; López-Ibáñez, S.; J. Villar, C.; Martins, C. B.; Ferreira, Joana D.; Assun 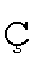 ão, M. F.G.; Santos, L. M.A.; Morais, J.; Castelo-Branco, R.; Reis, M. A.; Vasconcelos, V.; López-Ortiz, F.; Lomb6, F.; Soto, S. M. Microalgae and Cyanobacteria Strains as Producers of Lipids with Antibacterial and Antibiofilm Activity. Marine Drugs, 2021, 19, 675. https://doi.org/10.3390/md19120675
Yusim, A.; Utama, I. K. A. P., An Investigation Into The Drag Increase on Roughen Surface due to Marine Fouling Growth. IPTEK: The Journal for Technology and Science, 2017, 28.
Williamson, R.T.; Boulanger, A.; Vulpanovici, A.; Roberts, M.A.; Gerwick, W.H. Structure and absolute stereochemistry of phormidolide, a new toxic metabolite from the marine cyanobacterium Phormidium sp. J Org Chem., 2002, 15,67 (23), 7927-36. doi: 10.1021/jo020240s

## Claims

1. Novel compound phormidolide D (3) of formula:

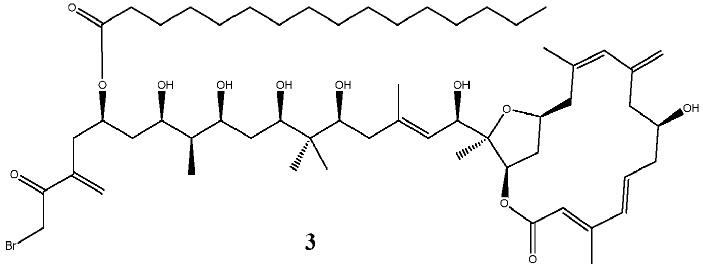

2. Compound phormidolide D (3) according to the previous claim wherein the compound is obtained from the organic extract of the cyanobacteria *Leptothoe sp. LEGE 181152.*

3. Compound phormidolide D (3) described in any of the previous claims for use in the treatment of cancer.

4. Compound phormidolide D (3) according to the previous claim, wherein the cancer is colorectal, breast, stomach, prostate, lung, or pancreatic cancer.

5. A composition comprising a therapeutically effective amount of compound phormidolide D (3) disclosed in any of the claims 1 to 2.

6. The composition according to the previous claim, for use in the treatment of cancer.

7. The composition according to the previous claim, wherein the cancer is colorectal, breast, stomach, prostate, lung, or pancreatic cancer.

8. Compound phormidolide D (3) described in any of the claims 1 to 2 for use as an antifouling agent.

9. An antifouling formulation comprising compound phormidolide D (3) described in any of the claims 1 to 2.

10. Antifouling formulation according to the previous claim for use in the reduction or prevention of macro- and microfouling marine organisms' attachment.

11. Bioactive compounds obtained from the organic extract of the cyanobacteria Leptothoe sp. LEGE 181152, wherein the compounds are hypoxanthine arabinoside (1) and 2'-deoxyinosine (2).

12. Bioactive compound hypoxanthine arabinoside (1) described in claim 11 for use as antifouling agent.

13. Bioactive compound 2'-deoxyinosine (2) described in claim 11 for use as antifouling agent.

14. An antifouling formulation comprising compound hypoxanthine arabinoside (1) or compound2'-deoxyinosine (2) described in claim 11.

15. Antifouling formulation according to the previous claim for use in the reduction or prevention of macro- and microfouling marine organisms.
